## Europäisches Patentamt

## European Patent Office

## Office européen des brevets

(19)

(11) Veröffentlichungsnummer: **0 073 941**

**A1**

(12) # EUROPÄISCHE PATENTANMELDUNG

(21) Anmeldenummer: 82107158.6

(22) Anmeldetag: 07.08.82

(51) Int. Cl.³: **C 07 C 21/06**
**C 07 C 17/34, C 07 C 17/38**

(30) Priorität: 05.09.81 DE 3135242

(43) Veröffentlichungstag der Anmeldung:
16.03.83 Patentblatt 83/11

(84) Benannte Vertragsstaaten:
BE DE FR GB IT NL SE

(71) Anmelder: DYNAMIT NOBEL AKTIENGESELLSCHAFT
Patentabteilung Postfach 1209
D-5210 Troisdorf, Bez. Köln(DE)

(72) Erfinder: Stephan, Rudolf, Dr.
Lessingstrasse 5
D-5210 Troisdorf-Sieglar(DE)

(72) Erfinder: Deselaers, Kurt, Dr.
Röntgenstrasse 2
D-5210 Troisdorf-Sieglar(DE)

(72) Erfinder: England, Gerhard
Porzer Strasse 32
D-5216 Niederkassel 3(DE)

(72) Erfinder: Oder, Manfred
Wildermannstrasse 10
D-5216 Niederkassel 3(DE)

(54) Verfahren zur Herstellung von Vinylchlorid.

(57) Vorliegende Erfindung befaßt sich mit verfahrenstechnischen Verbesserungen bei der Herstellung von Vinylchlorid durch thermische Spaltung von 1,2-Dichlorethan. Bei dieser Spaltung wurden die Nutzprodukte, nachdem sie einen Quenchturm bei Temperaturen um 130 °C verlassen hatten, bisher direkt auf 25 bis 30 °C abgekühlt und daraufhin destillativ aufgearbeitet. Erfindungsgemäß erfolgt die Abkühlung in zwei Stufen, wobei die Abkühlung in der ersten Stufe der Energiegewinnung dient. Bei dieser zweistufigen Abkühlung erfolgt jedoch eine zusätzliche Butadienbildung, die erfindungsgemäß dadurch behoben wird, daß man eisen- und chlorhaltiges 1,2-Dichlorethan demjenigen Dichlorethan-Strom zuführt, der als Rückfluß eine Schwersiederkolonne dient, in der der Sumpf des Quenchturms aufgearbeitet wird.

./...

Troisdorf, den 26. Aug. 1981
OZ: 81048 (4065) Dr.Sk/Ce

DYNAMIT NOBEL AKTIENGESELLSCHAFT
Troisdorf, Bez. Köln

Verfahren zur Herstellung von Vinylchlorid

Gegenstand der vorliegenden Erfindung ist ein kontinuierliches Verfahren zur Herstellung von Vinylchlorid, bei dem 1,2-Dichlorethan thermisch zu Vinylchlorid und Chlorwasserstoff zersetzt wird. Das vorliegende Verfahren betrifft eine teilweise Rückgewinnung der in den Prozeßgasen entstehenden Energie und eine Herabsetzung der dabei erhöht eintretenden Butadienbildung.

Es ist bekannt, Vinylchlorid durch thermische Spaltung von 1,2-Dichlorethan im Temperaturbereich zwischen 480 und 540 $^{o}$C zu gewinnen. Der dabei entstehende Chlorwasserstoff wird üblicherweise unter Zugabe von Ethylen und Sauerstoff in das als Einsatzprodukt für den Spaltprozeß dienende 1,2-Dichlorethan umgewandelt. Ein anderer Teil des 1,2-Dichlorethans kann z.B. durch Addition von elementarem Chlor an Ethylen in Gegenwart von Eisen-III-Chlorid herge-

- 2 -

stellt werden (Hydro Carbon Processing, Heft Nov. 79).

Bevor die den Spaltofen verlassenden Reaktionsprodukte ihrer destillativen Aufarbeitung zwecks Gewinnung der drei Nutzprodukte Chlorwasserstoff, Vinylchlorid und nicht umgesetztes 1,2-Dichlorethan zugeführt werden, müssen sie durch direkte oder indirekte Kühlverfahren möglichst schnell auf ein Temperaturniveau von etwa 25 bis 40 $^{o}$C gebracht werden, um die Bildung von Nebenprodukten zu vermeiden. Neben den oben erwähnten drei Hauptkomponenten führen die Ofengase jedoch auch Koks, teerhaltige Substanzen und Nebenprodukte wie Chloropren und Butadien mit sich. Die nicht destillierbaren Stoffe werden üblicherweise in einer dem Ofen nachgeschalteten Quenchkolonne abgetrennt und aus dieser z.B. über eine Schwersiederkolonne ausgeschleust.

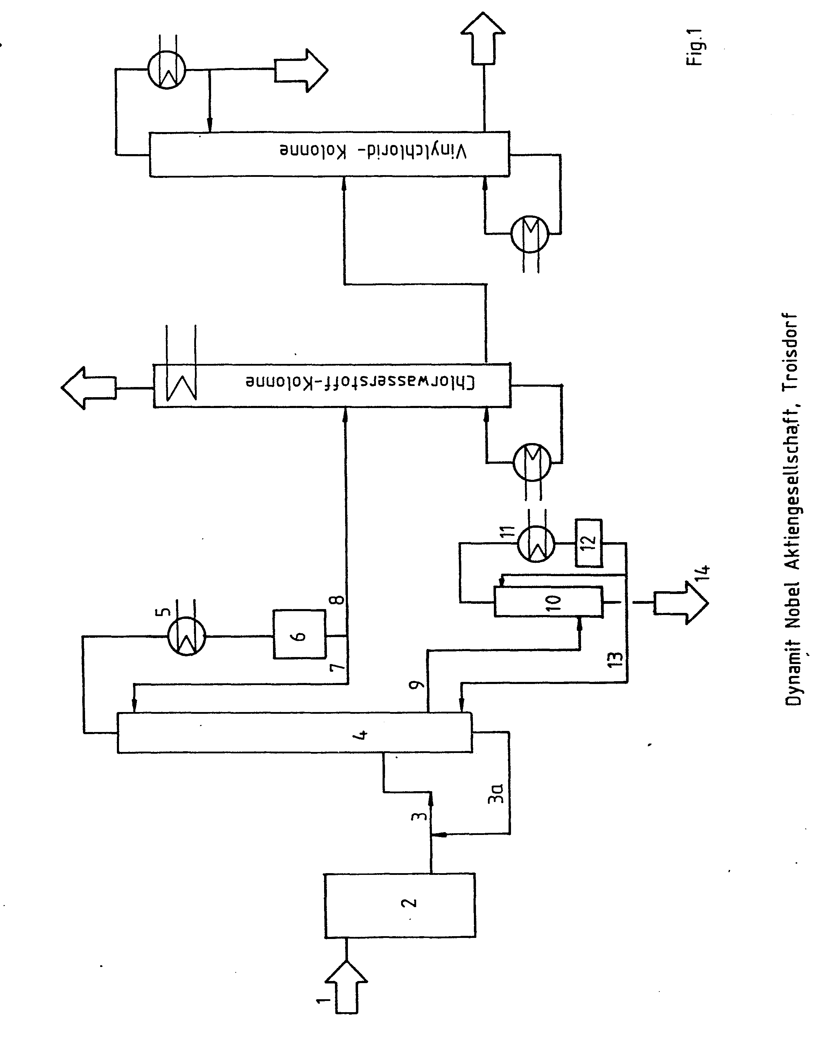

In Figur 1 ist das Verfahren schematisch dargestellt. Das über die Leitung 1 dem Ofen 2 zugeführte 1,2-Dichlorethan wird dort z.B. bei Temperaturen zwischen 480 und 550 $^{o}$C und einem Überdruck zwischen 5 und 20 bar teilweise zu Vinylchlorid und Chlorwasserstoff gespalten. Kurz hinter dem Ofen 2 werden in Leitung 3 die Reaktionsprodukte, welche die oben beschriebenen Verunreinigungen enthalten, durch bereits abgekühltes, nicht umgesetztes 1,2-Dichlorethan das auch noch andere, flüssige Hochsieder enthalten kann, aus der Quenchkolonne 4 über Leitung 3a auf etwa 160 $^{o}$C abgekühlt, indem dieses 1,2-Dichlorethan direkt in die Leitung 3 zwischen Ofen 2 und Quenchkolonne 4 eingespritzt wird. Das Quenchsystem dient einerseits der weiteren Abkühlung bzw. Kondensation der Ofengase, andererseits der Abscheidung von Teer, Koks und Hochsiedern. In dem indirekten Kühlsystem 5 werden die die Quenchkolonne 4 verlassenden Gase auf 25 bis 30 $^{o}$C gekühlt, wobei die kondensierten Substanzen im Sammler 6 aufgefangen und z.T. über Leitung 7 als Rückfluß der Quenchkolonne 4 zugeführt werden, während ein anderer Teil

- 3 -

zusammen mit den nicht kondensierten Substanzen über Leitung 8 in das sich anschließende Destillationssystem zur Auftrennung in die Einzelsubstanzen 1,2-Dichlorethan, Vinylchlorid und HCl gelangen. Aus dem Sumpf der Quenchkolonne 4 wird über Leitung 9 ein besonders Teer und Hochsieder enthaltender Produktstrom in eine Destillation 10 geleitet, in der die bis auf etwa 130 $^{\circ}$C unter Normaldruck siedenden Substanzen abdestilliert, im Kondensator 11 niedergeschlagen und über Sammler 12 und Leitung 13 zur Quenchkolonne zurückgeführt werden. Über Leitung 14 wird schließlich der Teer aus dem System entfernt.

Die beschriebene Verfahrensweise hat jedoch den Nachteil, daß im Kondensator 5 die in den Spaltgasen enthaltene Energie bei nicht mehr nutzbarem Temperaturniveau entweder an das als Kühlmittel dienende Wasser oder im Fall von Luftkühlern an die Atmosphäre verloren geht. Aufgabe der vorliegenden Erfindung war es deshalb, die Rückgewinnung dieser Energie bei möglichst geringem zusätzlichen Investitionsaufwand und unveränderter, möglichst verbesserter Qualität des Zielproduktes Vinylchlorid durchzuführen.

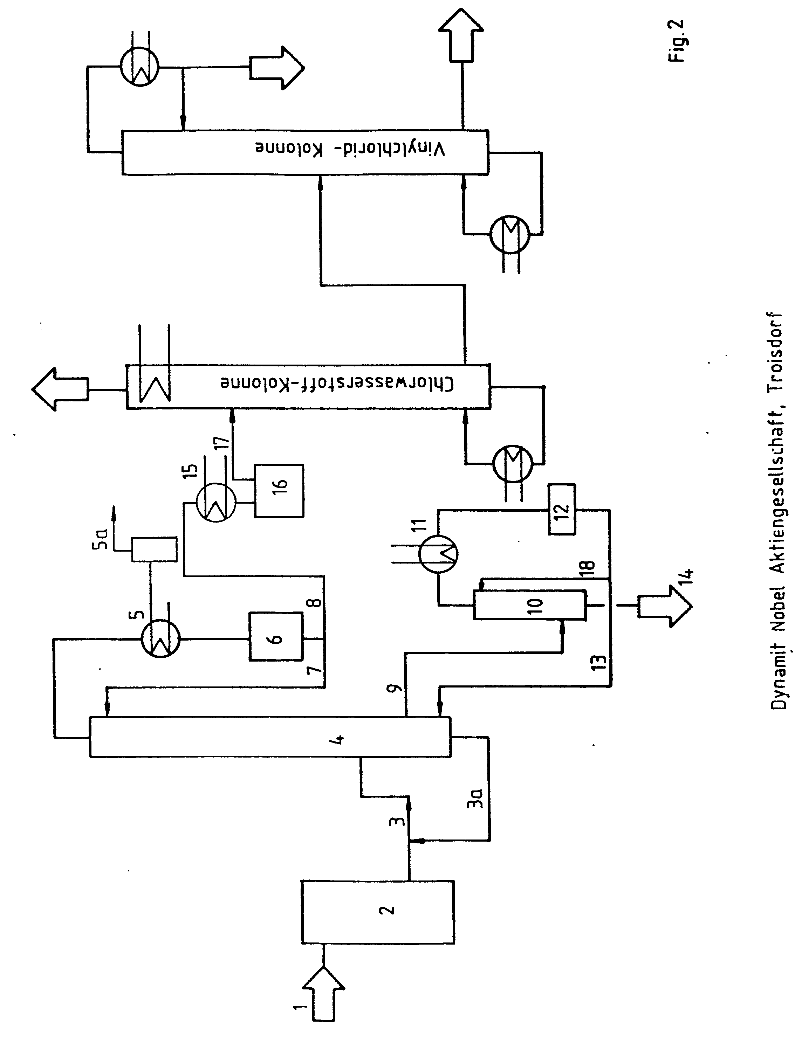

Die Lösung dieser Aufgabe erfolgt durch die im kennzeichnenden Teil des Anspruchs 1 genannten Maßnahmen. Erfindungsgemäß wird demzufolge die nach dem Quenchsystem 4 erforderliche Kühlung der gasförmigen Reaktionskomponenten in der Weise durchgeführt, daß die die Quenchkolonne bei einer Temperatur von 140 $^{\circ}$C verlassenden Spaltprodukte in einer Zwischenstufe zunächst auf 120 $^{\circ}$C gekühlt werden, wobei diese Zwischenstufe der Erzeugung von Prozeßdampf dient. Auf diese Weise können pro Tonne produziertem Vinylchlorid etwa 0,4 t Dampf von 1,7 bar abs. gewonnen werden. Dieser Dampf ist z.B. nutzbar zum Betreiben von Destillationskolonnen und Abwasserstrippern. Die Rückgewinnung eines Teils der Spaltenergie durch Erzeugung von Prozeßdampf auf

- 4 -

die beschriebene Weise hat gegenüber bekannten Verfahren (z.B. denjenigen, die in den DE-OS's 29 25 720 und 29 07 066 beschrieben sind) den Vorteil, daß durch diese Maßnahme die Laufzeit der Spaltanlage, z.B. durch Verstopfen von eventuell eingebauten energiegewinnenden Wärmeaustauschern unmittelbar hinter dem Spaltofen nicht eingeschränkt wird. Aus Figur 2 geht schematisch das neue Verfahren hervor.

Über Leitung 1 wird das 1,2-Dichlorethan dem Spaltofen 2 zugeführt. Bei 3 werden die etwa 500 $^\circ$C heißen unter einem Druck zwischen 5 und 15 bar stehenden Gase durch nicht umgesetztes, im Quenchsystem 4 abgekühltes und über Leitung 3a zurückgeführtes 1,2-Dichlorethan, das gegebenenfalls Hochsieder in Anteilen bis zu 15 Vol.-% enthalten kann, auf ein Temperaturniveau von 160 $^\circ$C gebracht. Wie in Figur 1, dient die Quenchkolonne 4 wiederum einerseits zum Abtrennen von Koks, teerartigen Substanzen und Hochsiedern, andererseits der Abkühlung und Kondensation der Ofengase. Die Wiedergewinnung der Spaltenergie erfolgt in der Weise, daß die die Quenchkolonne 4 mit einer Temperatur von 130 bis 140 $^\circ$C verlassenden Reaktionsprodukte einen Wärmeaustauscher 5 durchströmen, welcher kühlmittelseitig so mit Wasser betrieben wird, daß die Reaktionsprodukte nach Verlassen dieses Wärmeaustauschers 5 eine Temperatur zwischen 80 und 105 $^\circ$C besitzen. Bei geeigneter Dimensionierung des Wärmeaustauschers 5 dient somit die Energie der Spaltgase zur Erzeugung von Dampf. Über Leitung 5a kann dieser Dampf beliebigen Verbrauchern zugeführt werden.

Die im Wärmeaustauscher 5 kondensierten Bestandteile der Spaltgase werden in 6 gesammelt, dienen zum Teil über Leitung 7 als Rücklauf für die Quenchkolonne 4, zum anderen Teil gelangen sie über 8 in den Wärmeaustauscher 15. Die dort verflüssigten Komponenten werden im Sammler 16 aufgefangen und zusammen mit den nicht kondensierbaren Komponenten über Leitung 17 dem Aufarbeitungssystem zur Gewinnung von 1,2-Dichlorethan, Vinylchlorid und Chlorwasserstoff zugeführt.

Die Behandlung der in der Quenchkolonne 4 anfallenden Teersubstanzen und Hochsieder erfolgt über Leitung 9, wie in
Figur 1 bereits beschrieben.

Die Erzeugung von Dampf in der Zwischenstufe am Austauscher
5 hat zur Folge, daß das Kondensat im Sammler 6 mit einer
Temperatur von vorzugsweise etwa 100 $^{o}$C anfällt, während
das nach Figur 1 im Sammler 6 aufgefangene Kondensat auf
25 bis 35 $^{o}$C gekühlt wurde. Dieser Temperaturunterschied
des Kondensats aus dem Austauscher 5 führt nach vorliegender
Beobachtungen zu einer erhöhten Butadienbildung: Während bei
der Verfahrensweise nach Figur 1 der Butadiengehalt im
Vinylchlorid zwischen 6 und 10 ppm liegt, steigt er beim
Umschalten auf die Verfahrensweise gemäß Figur 2 mit Energierückgewinnung auf 15 bis 25 ppm an. Aus der Literatur
sind keine Zusammenhänge zwischen dem Butadiengehalt und
der Kondensationstemperatur der Spaltgase bekannt, jedoch
kann angenommen werden, daß bei höherer Temperatur unerwünschte Nebenreaktionen schneller ablaufen und zu Vorprodukten für die Butadienbildung führen.

Es wurde nun weiterhin gefunden, daß Vinylchlorid unter
teilweiser Rückgewinnung der Spaltenergie, gemäß Verfahrensweise nach Figur 2, mit geringem Butadiengehalt erzeugt werden kann, wenn man einen Strom von 1,2-Dichlorethan, welches z.B. in der sogenannten Direktchlorierung
aus Ethylen und Chlor in Gegenwart von Eisenchlorid gebildet wird, über die Leitung 18, Figur 2, in den Rückfluß der
Schwersiederkolonne 10 einleitet. Überraschenderweise sinkt
dabei der Butadienspiegel bei sonst unveränderten Bedingungen auf 3 bis 5 ppm. Ein aus der Direktchlorierung zugeführter 1,2-Dichlorethan-Strom enthält maximal 1400 ppm
Chlor und maximal 40 ppm Eisen; es kann jedoch auch ein auf

- 6 -

andere Weise hergestelltes Dichlorethan eingesetzt werden, sofern es den genannten Gehalt an Chlor und Eisenchlorid aufweist. Der beobachtete Rückgang des Butadienspiegels kann nicht einfach durch die bekannte Addition von Chlor an das Butadien oder die durch Eisen katalysierte Addition von Chlorwasserstoff an das Butadien erklärt werden: Die Hauptmenge des im Spaltofen gebildeten Butadiens reichert sich aufgrund dessen Siedepunktes nicht im Sumpf der Quenchkolonne 4 an; das Butadien kann nicht hauptsächlich über Leitung 9, Figur 2, in die Destillationseinheit 10 gelangen.

Durch die Zugabe des Eisenchlorid und Chlor enthaltenden Dichlorethans wird offenbar das Entstehen von Substanzen unterdrückt, die in der Schwersiederkolonne 10 zur Butadienbildung beitragen. Die für den gewünschten Effekt notwendige 1,2-Dichlorethanmenge ist außerordentlich gering. Ein Strom von 20 l/Std. genügt, um den Butadienspiegel von z.B. 15 auf 7 ppm zu senken. Die maximale notwendige Menge an Dichlorethan mit den angegebenen Chlor- und Eisengehalten liegt bei 1 Vol.-%, bezogen auf das Volumen des in den Spaltofen eingegebenen 1,2-Dichlorethans. Im allgemeinen genügen jedoch bereits 0,05 bis 0,2 Vol.-%, bezogen auf das Volumen des in den Spaltofen eingegebenen 1,2-Dichlorethans.

Der Eisen und Chlor enthaltende Dichlorethan-Strom wird erfindungsgemäß so eingeführt, daß das im 1,2-Dichlorethan gelöste Eisen nicht in den Hauptproduktstrom der Quenchkolonne gelangt. Die Anwesenheit von Eisen im gesamten Quenchsystem oder den nachgeschalteten Destillationskolonnen hätte zwei Nachteile: Einerseits ist bekannt, daß Eisen die Addition von Chlorwasserstoff an Vinylchlorid katalysiert, was bei vorliegendem Prozeß zu einer Ausbeuteminderung führen würde, andererseits führte ein erhöhter Eisenspiegel in den Umlaufverdampfern der Destillationskolonnen zu vermehrter Krack-Produktbildung, was die Laufzeit dieser Umlaufverdampfer nachteilig beeinflussen würde. Beide Nachteile treten nicht ein, wenn der zusätzlich zugeführte Eisen und Chlor enthaltende Dichlorethan-Strom über die Leitung 18 der Schwersiederkolonne 10 zugeführt wird.

Es wurde weiterhin gefunden, daß die oben beschriebene Zudosierung des Chlor und Eisen enthaltenden 1,2-Dichlorethan-Stroms für längere Zeit unterbrochen werden kann, ohne daß der Butadiengehalt im produzierten Vinylchlorid sofort wieder ansteigen würde. Es ist daher überraschenderweise möglich, mit der Dosierung des Dichlorethans zu beginnen, wenn ein gewünschter Butadienwert im Vinylchlorid überschritten wird, diese Dosierung nach deutlichem Absenken des Butadienwertes abzustellen und mit ihr erst wieder beim Erreichen eines vorgegebenen oberen Grenzwertes fortzufahren. Diese Arbeitsweise in Intervallen kann so geführt werden, daß die Phasen der Dichlorethan-Dosierung nur einige Stunden und die Phasen, in denen keine Dosierung nötig ist, einige Tage betragen. Diese Tatsache ist ein weiterer Beweis, daß die Senkung des Butadienspiegels im Vinylchlorid nicht durch einfache Addition von Chlor oder Chlorwasserstoff an das Butadien erklärt werden kann.

Beispiel 1(Vergleichsbeispiel) Figur 1

Es wurden 30 t/Std. 1,2-Dichlorethan unter einem Druck von 12 bar dem Spaltofen 2 zugeführt und die Temperatur während des Spaltvorgangs wird so gesteuert, daß die den Spaltofen verlassenden Reaktionsprodukte eine Temperatur von 525 $^{\circ}$C annehmen. Unmittelbar hinter dem Spaltofen werden die Reaktionsprodukte an der sogenannten Direkt-Quenchstelle 3

- 8 -

durch aus dem Quenchsystem 4, 5 und 6 zurückgeführtes, nicht umgesetztes 1,2-Dichlorethan auf 160 °C abgekühlt, bevor sie in die Quenchkolonne 4 eintreten.

Das aus dem Quenchturm 4 austretende Gasgemisch hat eine Temperatur von etwa 150 °C und gelangt in den mit Wasser von 23 °C betriebenen Wärmeaustauscher 5. Dort wird ein Gemisch von 1,2-Dichlorethan und Vinylchlorid kondensiert, das im Behälter 6 gesammelt wird. Das Kondensat hat eine Temperatur von 31 °C. Ein Teil dieses Kondensats wird in die Quenchkolonne 4 geführt, wo es als Rücklauf verwendet wird, während der andere Teil zusammen mit den nicht kondensierten Anteilen (z.B. Chlorwasserstoff) über Leitung 8 zur weiteren Aufarbeitung gelangt. Diese Aufarbeitung besteht im wesentlichen in einer destillativen Trennung des Gemischs Chlorwasserstoff, Vinylchlorid und 1,2-Dichlorethan.

Bei dieser Arbeitsweise werden 52 % des dem Spaltofen zugeführten 1,2-Dichlorethans in die Hauptprodukte Chlorwasserstoff und Vinylchlorid gespalten. Die in den Spaltgasen enthaltene Energie wird dabei praktisch vollständig an das Kühlwasser des Wärmeaustauschers 5 abgegeben. Aus dem Sumpf der Quenchkolonne 4 wird über Leitung 9 kontinuierlich ein Seitenstrom von 700 kg/Std. abgezogen. Dieser Strom besteht zu 83,9 % aus 1,2-Dichlorethan, 16,1 % aus Teer und Hochsiedern. In der Destillationseinheit 10 werden unter Normaldruck die bei 130 °C noch flüchtigen Substanzen, welche 1,2-Dichlorethan und geringe Mengen Vinylchlorid und HCl enthalten, verdampft, im Wärmeaustauscher 11 kondensiert, im Sammler 12 aufgefangen und über die Leitung 13 der Quenchkolonne 4 wieder zugeführt bzw. dienen als Rückfluß für die Schwersiederkolonne 10. Die oberhalb 130 °C siedenden Verbindungen wurden über die Leitung 14 aus dem System ausgeschleust. Der beschriebene Prozeß wurde über eine Laufzeit von 7 Monaten betrieben. Während dieser

- 9 -

Zeit stieg der Butadiengehalt des erzeugten Vinylchlorids von 6 auf 12 ppm an.

Beispiel 2  Figur 2

Die Verfahrensweise des Beispiels 1 wurde in gleicher Weise bis zum Eintritt der Spaltgase in die Quenchkolonne 4 durchgeführt. Mit einer Temperatur von 135 $^{o}$C treten die Spaltgase in den der Dampferzeugung dienenden Wärmeaustauscher 5 ein. Über Leitung 5a verlassen 4 t/Std. Dampf bei einem Druck von 1,7 bar absolut das System. Die in 5 kondensierten Komponenten werden in 6 gesammelt, erreichen dort eine Temperatur von 100 $^{o}$C und werden über Leitung 7 als Rücklauf bzw. Leitung 8 zusammen mit den nicht kondensierten Komponenten zum Wärmeaustauscher 15 weitergeführt. Hier erfolgt die Abkühlung von 100 auf 55 $^{o}$C im Sammler 16.

Die Hochsiederkolonne 10 wird betrieben wie im Beispiel 1 aufgezeigt wurde.

Nach 4 Monaten Laufzeit stieg der Butadienspiegel im erzeugten Vinylchlorid von 3 bis 5 ppm auf 20 bis 25 ppm an. Der Umsetzungsgrad des 1,2-Dichlorethans im Spaltreaktor war der gleiche wie im Beispiel 1.

Beispiel 3  Figur 2

Es wurde wie im Beispiel 2 gearbeitet. Zusätzlich wurden über Leitung 18 kontinuierlich 20 l Dichlorethan/h zugegeben, das einen Gehalt von 850 ppm Chlor und 2,8 ppm Eisen enthielt.

Vor der Zugabe des zusätzlichen Dichlorethans hatte das Vinylchlorid einen Butadiengehalt von 20 bis 25 ppm. Bereits nach 12 Stunden war der Butadiengehalt auf Werte zwischen 3 und 6 ppm gesunken. Diese Werte veränderten sich auch nach einer 4-monatigen Laufzeit bei angegebener Fahrweise nicht. Die übrigen Verfahrensergebnisse entsprachen denen des Beispiels 2.

<u>Beispiel 4  Figur 2</u>

Es wurde wie nach Beispiel 2 gearbeitet. Zusätzlich wurden über Leitung 18 während 24 Stunden 30 l Dichlorethan zugegeben, das einen Gehalt von 1080 ppm Chlor und 1,7 ppm Eisen enthielt. Vor der Zugabe hatte das Vinylchlorid einen Butadiengehalt von 20 bis 25 ppm. Nach 12 Stunden war der Butadiengehalt auf 3 bis 6 ppm abgesunken. Im Verlauf von 4 Tagen hielten sich die Butadienwerte auf gleichem Niveau und stiegen danach erst langsam wieder an. Bei einem Wert von 10 ppm wurde wieder mit der Dosierung des 1,2-Dichlorethanstroms begonnen. Nach 24 Stunden sank der Butadienspiegel wieder auf Werte zwischen 4 und 7 ppm. Diese Arbeitsweise konnte beliebig oft wiederholt werden, ohne daß ein vorgegebener Butadiengrenzwert von z.B. 10 ppm überschritten wurde. Die weiteren Verfahrensergebnisse entsprachen denjenigen des Beispiels 2.

Troisdorf, den 26. Aug. 1981
OZ: 81048 (4065) Dr.Sk/Ce

1 Patentansprüche:

1. Verfahren zur kontinuierlichen Herstellung von Vinylchlorid durch thermische Spaltung von 1,2-Dichlorethan, darauffolgendem Abkühlen der bei der Spaltung anfallenden Reaktionsprodukte auf Temperaturen zwischen 160 und 200 °C durch Einspritzen von 1,2-Dichlorethan, das aus dem nachgeschalteten Quenchturm abgeleitet wird, anschließendem Abscheiden der Hochsieder von den Nutzprodukten 1,2-Dichlorethan, Vinylchlorid und HCl in einem Quenchturm, Abkühlen der den Quenchturm bei Temperaturen zwischen 130 und 150 °C verlassenen Nutzprodukte auf 25 bis 40 °C und darauffolgender destillativer Trennung dieser Nutzprodukte in 1,2-Dichlorethan, Vinylchlorid und Chlorwasserstoff und teilweiser Rückführung der kondensierten Nutzprodukte auf den Kopf des Quenchturms, sowie destillativer Abtrennung von 1,2-Dichlorethan aus dem, die Hochsieder enthaltenden Sumpf des Quenchturms und Rückführung des abgetrennten Dichlorethans in den Quenchturm, dadurch gekennzeichnet, daß die den Quenchturm verlassenden Gase in einer ersten Stufe auf Temperaturen zwischen 90 und 110 °C unter Erzeugung von Prozeßdampf und in einer zweiten Stufe auf 40 bis 60 °C abgekühlt werden und daß das aus den Hochsiedern abgetrennte Dichlorethan, welches zum Teil als Rückfluß auf die Schwersiederkolonne geführt wird, mit einem zusätzlichen Dichlorethanstrom versetzt wird, der maximal 1 % der ursprünglich in den Spaltofen eingeführten 1,2-Dichlorethanmenge beträgt, und welcher Chlor und Eisenchlorid in einer maximalen Menge von 1400 ppm Chlor und 40 ppm Eisen enthält.

2. Verfahren gemäß Anspruch 1, dadurch gekennzeichnet, daß die zusätzlich zugeführte Dichlorethanmenge diskontinuierlich in zeitlichen Intervallen hinzugeführt wird.

**0073941**

Fig.1

Dynamit Nobel Aktiengesellschaft, Troisdorf

**0073941**

Fig.2

Dynamit Nobel Aktiengesellschaft, Troisdorf

Europäisches
Patentamt

**EUROPÄISCHER RECHERCHENBERICHT**

**0073941**
Nummer der Anmeldung

EP 82 10 7158

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| | --- | | C 07 C 21/06 |
| A | EP-A-0 021 381 (UHDE; HOECHST) | | C 07 C 17/34 |
| | | | C 07 C 17/38 |
| | --- | | |
| A,P | DE-A-3 013 107 (HOECHST) | | |
| | ----- | | |

RECHERCHIERTE
SACHGEBIETE (Int. Cl. ³)

C 07 C 21/00
C 07 C 17/00

Der vorliegende Recherchenbericht wurde für alle Patentansprüche erstellt.

| Recherchenort | Abschlußdatum der Recherche | Prüfer |
|---|---|---|
| DEN HAAG | 02-12-1982 | VAN GEYT J.J.A. |

EPA Form 1503. 03.82